# FASCICULE DE BREVET EUROPEEN

(11) **EP 0 777 691 B1**
(45) Date de publication et mention de la délivrance du brevet: **02.05.2001**
(21) Numéro de dépôt: 96922960.8
(22) Date de dépôt: 20.06.1996
(51) Int. Cl.: C08F 2/44

(54) **PROCEDE DE PREPARATION PAR POLYMERISATION EN DISPERSION D'UN LATEX MONODISPERSE CALIBRE**
POLYMERISATIONSVERFAHREN ZUR HERSTELLUNG VON KALIBRIERTEM MONODISPERSEM LATEX
POLYMERISATION PROCESS FOR THE PREPARATION OF CALIBRATED MONODISPERSE LATEX IN DISPERSION

(30) Priorité: 22.06.1995 FR 9507486
(43) Date de publication de la demande: 11.06.1997
(73) Titulaire: MERCK EUROLAB S.A., 94120 Fontenay-sous-Bois (FR)
(72) Inventeur: RICHARD, Joel, F-49160 Longue (FR); VASLIN, Sophie, F-94360 Bry-sur-Marne (FR)
(74) Mandataire: Ahner, Francis
(86) Numéro de dépôt international: FR9600957
(87) Numéro de publication internationale: WO9700896

(56) Documents cités:
- EP-A- 0 038 730

## Description

La présente invention concerne un procédé de préparation en dispersion d'un latex magnétique monodisperse calibré.

Lesdits latex trouvent une application en biologie, notamment dans les méthodes de diagnostics, en chromatographie d'affinité, comme support pour les sondes à acides nucléiques, et en chimie .

Les latex magnétiques sont des dispersions aqueuses de microsphères comprenant une matrice de polymère et des charges magnétisables qui confèrent à ces microsphères des propriétés magnétiques, notamment superparamagnétiques.

On rappellera brièvement qu'à côté des charges paramagnétiques et ferromagnétiques, l'on trouve une troisième catégorie de charges appelées charges superparamagnétiques qui sont caractérisées par le fait qu'elles ne restent pas magnétiques en l'absence d'un champ magnétique et que ces charges ont une aimantation magnétique presque aussi élevée que celle des charges ferromagnétiques.

Il s'agit le plus souvent d'oxydes de fer tels que la magnétite, l'hématite, la ferrite lorsqu'elles possèdent une granulométrie inférieure à une certaine limite critique.

Sous l'influence d'un champ magnétique, les microsphères magnétisables sont attirées vers l'aimant et peuvent de ce fait être rapidement séparées de la phase aqueuse.

Cette séparation est réversible puisqu'en l'absence de champ magnétique, les microsphères perdent leur aimantation et se redispersent spontanément. La taille des microsphères est généralement comprise entre quelques dizaines de nanomètres et plusieurs microns.

La grande surface spécifique conférée par leur faible taille peut être exploitée avantageusement dans les applications de type immunologique mettant en oeuvre la réaction anticorps antigène. Le latex magnétique joue le rôle de support qui va permettre le dosage quantitatif des espèces présentes dans le milieu après séparation.

Les étapes de séparation et de lavage sont dans ce cas grandement simplifiées grâce à l'utilisation de ces microsphères magnétisables.

A ce jour, les procédés exploités, notamment ceux conduisant aux latex magnétiques commercialisés sous le nom de marque ESTAPOR^{®}, reposent sur les mécanismes de polymérisation en microsuspension.

Le brevet US 4 339 337 permet de préparer des microsphères magnétisables de diamètre allant de 0,05 à 3 millimètres par polymérisation en suspension d'un monomère vinylaromatique en présence d'un amorceur organosoluble, d'un agent de suspension et d'une charge magnétisable dispersée au sein d'une solution dans ledit monomère d'un polymère non hydrosoluble. Les microsphères obtenues renferment des charges magnétisables réparties dans la matrice de polymère.

On a également proposé par le brevet US 4 358 388 de préparer des latex de polymères hydrophobes magnétisables par homogénéisation d'une solution aqueuse d'émulsifiant et d'une dispersion d'une charge magnétisable dans une phase organique formée d'un amorceur organosoluble, de tout ou partie du monomère hydrophobe et/ou d'un composé organique insoluble dans l'eau, puis polymérisation. Les latex obtenus sont formés de microsphères de polymères de diamètre d'environ 0,03 à 5 micromètres renfermant des charges magnétisables réparties dans la matrice de polymère.

Dans la polymérisation en suspension, on disperse le monomère sous forme de gouttelettes (taille comprise entre 100 et 10 000 micromètres et 0,05 à 5 micromètres dans la microsuspension) dans la phase aqueuse. L'initiateur est soluble dans la phase organique et la suspension est maintenue par agitation mécanique et addition de stabilisant (colloïde organique ou minéral dans le cas de la suspension classique, émulsifiant en microsuspension). Chaque gouttelette de monomère est considérée comme un mini réacteur, dans lequel la cinétique de polymérisation est celle de la polymérisation en masse. Elle permet d'obtenir des microsphères avec une distribution granulométrique relativement large en suspension classique et un latex très polydisperse dans le cas de la microsuspension.

Le brevet US 5 242 964 décrit un procédé permettant d'obtenir un latex monodisperse à partir du latex obtenu par le brevet US 4 358 388 (correspondant au brevet EP-A-0 038 730) par ajout d'un surfactant puis séparation des deux phases formées et récupération de la phase solide.

Les latex obtenus par les procédés décrits dans l'art antérieur conduisent par conséquent à des distributions granulométriques très larges (typiquement de quelques dizaines de microns à plusieurs microns), sauf à effectuer des étapes supplémentaires. Cependant, l'efficacité de l'utilisation des microsphères magnétisables en tant que support de réactif immunologique est d'autant meilleure que la séparation magnétique sera rapide. Cette rapidité est directement fonction de l'homogénéité des microsphères et en particulier elle augmente lorsque leur distribution granulométrique se resserre.

Des microsphères magnétiques monodisperses calibrées permettraient d'obtenir des séparations de phase homogène et rapide. De plus la monodispersité, et la calibration en taille permettraient d'accéder exactement à la surface réelle d'adsorption des microsphères et donc à la capacité de fixation théorique de celles-ci vis-à-vis des espèces réactives.

Un objet de la présente invention est de proposer un procédé de préparation de latex de microsphères magnétisables simple et rapide conduisant en une seule étape à des latex de microsphères magnétisables et calibrées présentant une taille comprise entre 0,5 et 5 micromètres.

En premier lieu, l'invention concerne un procédé de préparation par polymérisation en dispersion d'un latex magnétique monodisperse calibré forme d'une dispersion aqueuse de microsphères magnétisables constituées d'une matrice de polymères hydrophobes dérivés d'au moins un monomère vinylique et de charges magnétisables, lesdites microsphères possédant une distribution granulométrique calibrée, une taille comprise entre 0,5 et 5 micromètres, caractérisé en ce que l'on fait réagir en une seule étape une solution organique comprenant au moins un monomère vinylique polymérisable, un solvant organique, qui est solvant pour les monomères et non solvant pour le polymère formé, des charges magnétisables sous forme de ferrofluide, un système stabilisant pour le polymère formé, en présence d'un initiateur de polymérisation organosoluble et en ce que l'on récupère ledit latex magnétique.

Par le terme "matrice de polymère", on entend que les charges magnétisables sont au moins partiellement noyées dans cette matrice, la partie résiduelle pouvant être située à la surface de la microsphère.

Par l'expression "distribution granulométrique calibrée", on entend que cette distribution présente un écart-type inférieur ou égal à 15%, de préférence inférieur ou égal à 10 % et avantageusement inférieur ou égal à 5 % ; ceci signifie que les 2/3 en poids des microsphères ont un diamètre compris entre dₘ-σ et dₘ+σ (dₘ : diamètre moyen, σ : écart-type), dans le cas d'une distribution du type de Gauss.

Plusieurs autres critères structuraux définissent également les microsphères obtenues par le procédé selon l'invention.

Les microsphères comprennent un colloïde protecteur, comme cela sera décrit ci-après.

La présence d'un colloïde protecteur en surface et/ou dans l'épaisseur de la matrice de polymère confère aux microsphères une stabilité accrue aux forces ioniques élevées (stabilisation stérique et éventuellement électrostatique).

Par ailleurs, à dimension égale, elles sédimentent plus lentement que les microsphères obtenues par des méthodes de polymérisation en suspension ou en émulsion.

Le procédé de polymérisation en dispersion permet d'obtenir en outre, des matrices de polymère formant des microsphères dont les chaînes ont en général une masse moléculaire moyenne (en poids, en nombre, en Z), inférieure à celle des chaînes obtenues par d'autres procédés, que ce soit en émulsion ou en suspension.

Un procédé de polymérisation en dispersion particulièrement avantageux sera décrit ci-après.

Les charges magnétisables comprennent de préférence une couche superficielle, le plus souvent monomoléculaire, d'un agent dispersant non hydrosoluble.

Ces charges se présentent donc sous la forme d'un fluide magnétique appelé "ferrofluide", cf. Kaiser and Miskolczy, J. Appl. Phys. 41 3 1064 (1970). Un ferrofluide est une dispersion de charges magnétiques très fines préservées de toute agglomération par le mouvement Brownien.

Pour empêcher toute agglomération des charges du fait des forces attractives de Van der Waals, les charges sont revêtues comme indiqué précédemment. Quand un champ magnétique est appliqué, la force magnétique est transmise au volume entier de liquide et le ferrofluide répond comme un fluide, à savoir que des charges magnétiques ne se séparent pas de leur milieu.

Parmi les agents dispersants formant un revêtement mono-moléculaire non hydrosoluble autour des charges magnétisables, on peut citer ceux présentant une longue chaîne hydrocarbonée terminée par un groupe polaire du type -COOH, NH₂ ..., tels que les acides gras, les amines grasses .... contenant au moins 12 atomes de carbone et plus particulièrement les acides gras en C₁₈, tels que les acides oléique, linoléïque et linolénique.

Les charges magnétisables revêtues d'agents dispersants peuvent être préparées par exemple par peptisation dans l'agent dispersant de charges magnétiques obtenues par voie sol-gel, dispersion dans un liquide vecteur organique (brevet US 3 843 540) puis floculation à l'aide d'un solvant polaire du type cétone, ester, alcool et séparation des charges enrobées.

Par l'expression "monomère vinylique", on entend tous les monomères possédant une double liaison éthylénique pouvant permettre la polymérisation. Il s'agit donc de monomères vinylaromatiques parmi lesquels on peut citer le styrène, l'alpha-méthylstyrène, l'éthylstyrène, l'éthylvinylbenzène, le tertiobutylstyrène, le vinyltoluène, le chloroslyrène, le bromostyrène, le divinylbenzène. Il s'agira également des monomères vinyliques présentant un groupement attracteur tel que l'acétate de vinyle, le chlorure de vinyle, ou des dérivés acryliques tels que les acrylates ou méthacrylates notamment d'alkyle en C₁-C₁₂ éventuellement hydroxylés comme les hydroxyalkylacrylates ou méthacrylates, l'acide acrylique ou méthacrylique ou les amides de ceux-ci comme l'acrylamide, les dérivés diacryliques ou leurs esters ou leurs anhydrides comme l'anhydride maléique. On pourra utiliser également, notamment à titre de comonomères des diènes tels que l'isoprène, le butadiène. Les chaînes de polymères sont des homopolymères, copolymères ou terpolymères dérivés de ces monomères vinyliques, statistiques ou séquencés.

De préférence, les charges magnétisables représentent 0,5 à 50 % environ en poids du poids desdites microsphères magnétisables. Avantageusement, cette proportion sera supérieure à 10 %.

Les microsphères magnétisables obtenues ont une taille comprise entre environ 0,5 et 5 micromètres.

De préférence, le monomère est choisi dans le groupe constitué par le styrène ou les dérivés du styrène, les acrylates ou méthacrylates d'alkyle, (les radicaux alkyle ont de préférence entre 1 et 10 atomes de carbone avantageusement 1 à 5), l'acide acrylique ou méthacrylique, le chlorure de vinyle, l'acétate de vinyle, le butadiène, l'isoprène.

Avantageusement le polymère obtenu est choisi dans le groupe constitué par le polystyrène, le polyméthacrylate d'alkyle ou le polyacrylate d'alkyle ou des copolymères statistiques ou séquencés de styrène et d'acrylate ou méthacrylate d'alkyle.

La polymérisation en dispersion est un procédé bien connu, simple et en une seule étape, permettant de préparer des microsphères de latex monodisperses. Cette technique se différencie de la polymérisation en suspension ou microsuspension par l'homogénéité initiale du milieu de polymérisation. La caractéristique distinctive de la polymérisation en dispersion est due au fait que l'on choisit le milieu solvant et la composition du mélange monomère*/*solvant de telle sorte que le monomère soit soluble dans le milieu solvant alors que le polymère formé ne l'est pas.

En d'autres termes, le monomère est soluble dans le milieu réactionnel non aqueux alors que le polymère est insoluble.

L'initiateur est également soluble dans le milieu et afin de stabiliser les microsphères de polymères formées, on utilise un système de stabilisation stérique chargée ou non (colloïde protecteur).

Schématiquement, on peut donner une représentation du mécanisme de polymérisation en dispersion sans que cette représentation puisse en aucune manière limiter la portée de la présente invention

Au début de la polymérisation, le monomère, le système stabilisant et l'initiateur forment avec le milieu dispersant une solution homogène qui constitue la phase continue.

L'initiateur est décomposé thermiquement et les radicaux libres formés réagissent avec le monomère en solution pour former des radicaux oligomères solubles en croissance.

Lorsque les oligomères ont atteint une longueur de chaîne critique, ils précipitent pour former des microsphères primaires stabilisées par adsorption et/ou greffage du colloïde protecteur. Ces nucléi formés absorbent le monomère à partir de la phase continue, et la polymérisation se poursuit alors dans les microsphères gonflées de monomères jusqu'à épuisement de ce dernier.

Par certains aspects, ce type de polymérisation s'apparente à la fois à la polymérisation en émulsion avec formation de nucléi, suite à la précipitation de chaînes en croissance dans la phase homogène (mécanisme de Fitch), et à la polymérisation en solution avec un amorceur organosoluble.

Cette technique permet de préparer en une seule étape, et très simplement, des microsphères monodisperses calibrées jusqu'à 5 micromètres de diamètre grâce à un ajustement judicieux des conditions opératoires (température, stabilisant, initiateur ...)..

Pour arriver à des microsphères finales monodisperses calibrées, il faut remplir les conditions suivantes :
- l'étape de nucléation doit être rapide de façon à ce que tous les nucléi se forment simultanément.
- tous les radicaux oligomères générés dans la phase continue durant la période de croissance des microsphères, doivent être capturés par les microsphères existantes avant d'atteindre la taille critique de précipitation, ce qui donnerait lieu à la formation de nouvelles microsphères,
- la coalescence entre les microsphères durant la phase de croissance doit être évitée.

Selon un mode de réalisation, on dissout l'initiateur dans le monomère et on ajoute le ferrofluide (solution A). On dissout ensuite le système stabilisant dans le solvant (solution B). La solution B est chauffée à une température d'environ 40 à 80°C, généralement aux alentours de 60°C, puis après une durée déterminée, on ajoute la solution A.

La polymérisation est effectuée pendant une durée de plusieurs heures selon le mélange de départ.

Les microsphères sont ensuite redispersées dans l'eau après application d'un champ magnétique puis remplacement du surnageant par de l'eau distillée additionnée éventuellement de 0,5 % de SDS (laurylsulfate de sodium).

Les microsphères sont caractérisées par leur diamètre, leur taux de fer et dans le cas où le dispersant est à base d'un acide gras par le nombre de fonctions carboxyles.

Le choix du solvant dépend de la nature du ferrofluide, du monomère ainsi que des autres réactifs de manière à ce que le solvant devienne non solvant du polymère dès que celui-ci atteint une taille critique.

De plus, lors de la précipitation des oligomères, la matière magnétisable ne doit pas être exclue de la phase dispersée et doit se localiser au sein des microsphères.

Parmi les solvants polaires, on citera notamment les alcools ou mélanges d'alcools comme le méthanol, l'éthanol, l'isopropanol, le butanol, le 2-méthylpropanol, les éthers alkyliques comme le diéthyléther ou le méthyléthyléther ou les mélanges alcool-eau ou éther-alcool.

Parmi les solvants apolaires, on citera notamment les solvants hydrocarbonés aliphatiques comme le cyclohexane, l'hexane.

Les solvants polaires sont préférés aux solvants apolaires, car ils permettent d'utiliser une gamme beaucoup plus large de monomères vinyliques.

De préférence, lorsque le solvant est un solvant polaire, on utilisera seuls ou en mélange les monomères choisis dans le groupe constitué par les monomères vinylaromatiques tels que le styrène, les dérivés du styrène (vinyltoluène, éthylvinylbenzène, bromostyrène, chlorostyrène, alphaméthylstyrène, et éthylstyrène), les dérivés acryliques tels que l'acide acrylique, méthacrylique, les acrylates et méthacrylates d'alkyle dont le groupe alkyle possède de un à dix atomes de carbone, les hydroxyalkylacrylates ou méthacrylates, l'acrylamide ou le méthacrylamide, qui permettent de fonctionnaliser la surface des microsphères et d'ajuster leur hydrophilie, les monomères vinyliques présentant un groupement attracteur tels que les esters d'acide éthylénique à 4 ou 5 atomes de carbone, le chlorure de vinyle, les diènes, comme le butadiène, l'isoprène.

Selon une variante avantageuse, l'invention concerne un latex dont les microsphères sont fonctionnalisées par des groupes de surface parmi lesquels on peut citer les radicaux carboxyle, amino, chloro, chlorométhyle, sulfonate, sulfate, hydroxy, thiol.

Dans ce cas, le colloïde protecteur est avantageusement un polymère choisi dans le groupe constitué par la poly(vinyl pyrrolidone), les homopolymères acryliques ou méthacryliques, les copolymères statistiques ou séquencés de monomères choisis dans le groupe constitué par le styrène ou les dérivés du styrène, le butadiène d'une part, l'acide méthacrylique, l'acide acrylique d'autre part, les dérivés cellulosiques comme l'hydroxypropylcellulose, les polyéthylènes oxyde, les monoesters du polymère de l'anhydride maléique, le polymère de l'acide maléique. De préférence, il s'agit de la poly(vinyl pyrrolidone) ou d'un polyacrylate ou méthacrylate. Généralement, le colloïde protecteur contribue également à l'introduction de groupes fonctionnalisés à la surface des microsphères.

Lorsque le solvant est un solvant apolaire, notamment les solvants hydrocarbonés comme l'hexane ou le cylohexane, les monomères sont choisis notamment dans le groupe constitué par les acrylates d'alkyle, méthacrylates d'alkyle en C₁-C₁₂ et les dérivés styréniques, les diènes.

Le système stabilisant est un polymère choisi dans le groupe constitué par les polyacrylates ou méthacrylates, les copolymères séquencés ou statistiques de monomères choisis dans le groupe constitué par les oléfines telles que l'isobutylène, le butylène, les dérivés styrèniques, le butadiène, les dérivés du butadiène comme l'isoprène, les esters vinyliques d'une part, les dérivés acrylates ou méthacrylates, les alkylsiloxanes d'autre part. On peut notamment utiliser des copolymères séquencés styrène-butadiène-styrène ou styrène-butadiène.

L'initiateur de polymérisation est de préférence choisi dans le groupe constitué par l'AIBN ou 2,2'-azobis(isobutyronitrile), l'ACPA ou 4,4'-azobis(acide 4-cyanopentanoique), l'AMBN ou 2,2'-azobis(2-méthylbutyronitrile), le BPO ou peroxyde de benzoyle, l'ADVN ou 2,2'-azobis(2,4-diméthylvaleronitrile).

Dans tous les cas, de préférence, la solution comprend un costabilisant ionique tel qu'un dioctylsulfosuccinate de sodium (Aérosol OT^{®}). D'autres costabilisants peuvent également convenir, comme le chlorure de méthyltricaprylammonium.

On peut également ajouter dans le milieu réactionnel (latex) des additifs colloïdaux comme par exemple de la silice, afin de diminuer la viscosité du milieu.

Selon une variante préférée, le procédé de préparation est caractérisé en ce que la solution comprend en partie en poids :

| | |
|---|---|
| solvant organique | 45 à 90 |
| monomère | 5 à 30 |
| ferrofluide | 2 à 30 |
| système stabilisant | 2 à 10 |
| costabilisant | 0,1 à 1 |
| initiateur | 0,1 à 1 |

Par les fonctions éventuellement présentes sur le colloïde protecteur, les microsphères magnétisables présentent l'avantage d'être fonctionnalisées de manière connue. Les groupemements fonctionnels polaires avec ou sans groupe réactif facilitent l'association subséquente avec des molécules biologiques telles que les anticorps. Dans le cas où les groupements fonctionnels présentent un groupe réactif, on parlera de greffage avec la molécule d'intérêt, notamment biologique.

Par ailleurs, les protéines peuvent être également fixées sur la surface soit par adsorption passive ou de préférence par liaison covalente au moyen de groupes carboxyles lorsque ceux-ci sont présents.

Les latex trouvent une application dans le domaine de la chimie ou de la biologie, notamment pour les méthodes de diagnostique, de séparation, de calibration. C'est le cas notamment des méthodes de dosage biologique bien connues de l'homme du métier, pour fixer ou immobiliser des substances biologiquement actives (protéines, anticorps, enzymes, antigènes, médicaments) par adsorption ou couplage.

Ces microsphères peuvent être utilisées à titre de support dans les tests de diagnostique (agglutination "RIA" ou en anglais radioimunological assay - "IRMA" ou en anglais immunoradiometric assay - "EIA" ou en anglais enzyme immuno assay - "ELISA" enzyme linked immunosorbent assay) ou pour les sondes d'acides nucléiques, comme catalyseur enzymatique en biotechnologie ou comme support de culture cellulaire.

Bien entendu compte tenu de leur taille très réduite, les microsphères et les dispersions aqueuses correspondantes sont également susceptibles d'être employées avantageusement dans des domaines plus industriels, de type industrie de revêtement comme la peinture, l'encollage, le textile, le papier ...

L'invention est maintenant illustrée par les exemples suivants décrits à titre illustratif seulement. Le mode opératoire général commun à tous ces exemples fait intervenir les réactifs suivants :
solvant alcoolique organique,
monomère(s)
poly(vinyl pyrrolidone),
dioctylsulfosuccinate de sodium,
ferrofluide
2,2'-azobis(isobutyronitrile) en tant qu'initiateur.

Les conditions opératoires répondent aux caractéristiques suivantes :
réaction en atmosphère d'azote à une température de polymérisation de 70° pendant plusieurs heures, environ 20 heures.

En premier lieu, on dissout l'initiateur dans le ou les monomères (solution A), on agite et on ajoute ensuite le ferrofluide.

On dissout la poly(vinyl pyrrolidone) et le dioctylsulfosuccinate de sodium dans le solvant et l'on agite pendant plusieurs heures (solution B).

On introduit la solution B dans un réacteur, on chauffe à 60°C pendant 40 minutes, on maintient cette température pendant plusieurs minutes et l'on ajoute la solution A.

La température augmente jusqu'à 70°C et la polymérisation s'effectue pendant la durée indiquée ci-dessus.

Les microsphères sont ensuite remises en suspension après séparation par action d'un champ magnétique puis remplacement du surnageant par de l'eau distillée additionnée de 0,5 % de SDS.

Les latex obtenus dans les exemples ci-après ont été caractérisés du point de vue granulométrie à l'aide du granulomètre laser CILAS 850 commercialisé par CILAS et du point de vue détermination des fonctions de surface par dosage conductimétrique des groupements acides.

### Exemple 1

La solution comprend en poids :

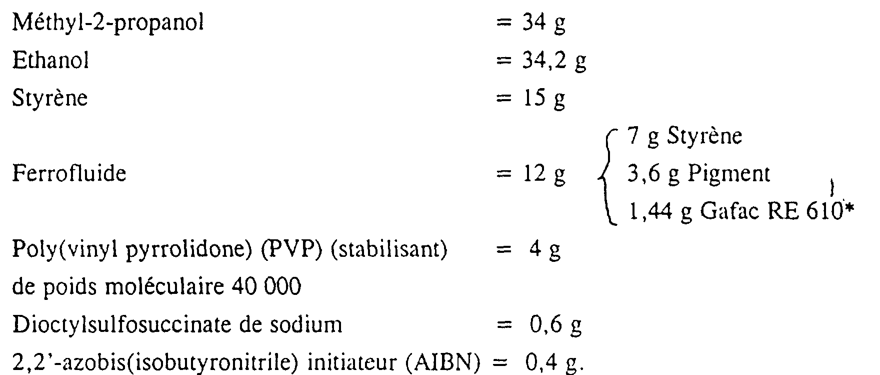

Le Gafac RE 610* est un mélange de mono et diesters phosphoriques de dérivés alkyl-aryl éthoxylés, commercialisé par Rhône-Poulenc.

Les mesures effectuées montrent que les microsphères ont un diamètre moyen de 0,87 micromètre avec un écart-type de 15 %.

Le taux de fer est de 11,2 % et le nombre de fonctions carboxyles est de 0,025 milliéquivalents molaires (25 microéquivalents) par gramme de microsphères sèches.

### Exemple 2

On réalise la solution suivante :

| | |
|---|---|
| Méthyl-2-propanol | = 34 g |
| Ethanol | = 34,3 g |
| Styrène | = 22 g |
| Ferrofluide | = 5 g |
| PVP de poids moléculaire 40 000 | = 4 g |
| Dioctylsulfosuccinate de sodium | = 0,3 g |
| AIBN | = 0,38 g. |

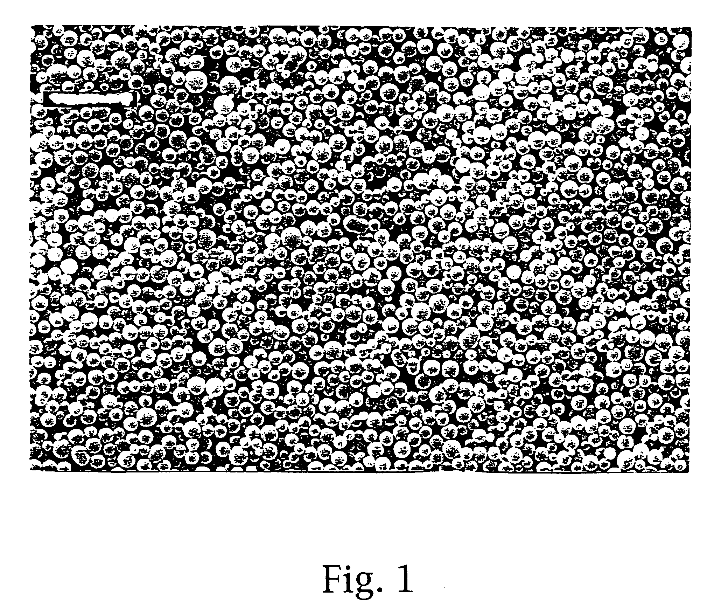

Les mesures effectuées montrent que les microsphères ont un diamètre moyen de 1,07 micromètre avec un écart-type de 15 %. Le taux de fer est de 4,7 %. Une photographie ci-jointe pour un agrandissement de 5000 fois montre l'homogénéité remarquable des microsphères obtenues (figure 1).

### Exemple 3

On réalise la solution suivante :

| | |
|---|---|
| Méthyl-2-propanol | = 34g |
| Ethanol | = 34 g |
| Styrène | = 7 g |
| Ferrofluide | = 20 g |
| PVP de poids moléculaire 40 000 | = 4 g |
| Dioctylsulfosuccinate de sodium | = 0,3 g |
| AIBN | = 0,38 g. |

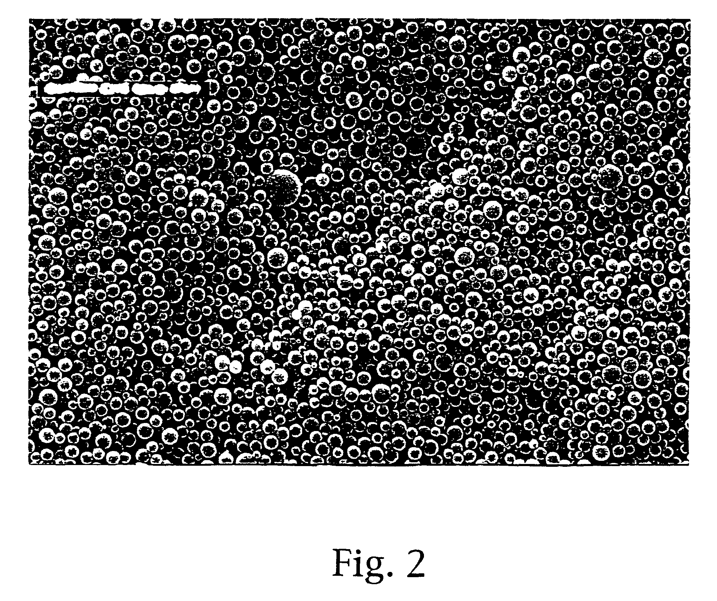

Les mesures réalisées montrent que les microsphères ont un diamètre moyen de 1,15 micromètre avec un écart-type de 10 %. Le taux de fer est de 19 %. La photographie ci-jointe pour un agrandissement de 5000 fois montre l'homogénéité remarquable des microsphères obtenues (figure 2).

### Exemple 4

On réalise la solution suivante :

| | |
|---|---|
| Méthyl-2-propanol | = 34 g |
| Ethanol | = 34 g |
| Styrène | = 15 g |
| Ferrofluide | = 12 g |
| PVP de poids moléculaire 360 000 | = 2,5 g |
| Dioctylsulfosuccinate de sodium | = 0,3 g |
| AIBN | = 0,4 g. |

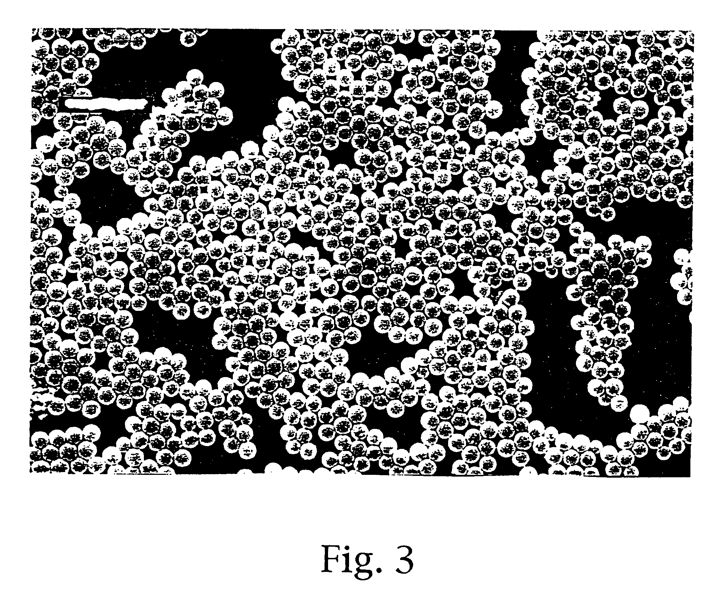

Les mesures effectuées montrent que les microsphères ont un diamètre moyen de 1,3 micromètre avec un écart-type de 5 %. Le taux de fer est de 12 %. Une photographie ci-jointe pour un agrandissement de 5000 fois montre l'homogénéité remarquable des microsphères obtenues (figure 3).

### Exemple 5

On réalise la solution suivante :

| | |
|---|---|
| Méthyl-2-propanol | = 34 g |
| Ethanol | = 34 g |
| Styrène | = 15 g |
| Ferrofluide | = 12 g |
| PVP de poids moléculaire 360 000 | = 1,5 g |
| Dioctylsulfosuccinate de sodium | = 0,3 g |
| AIBN | = 0,4 g. |

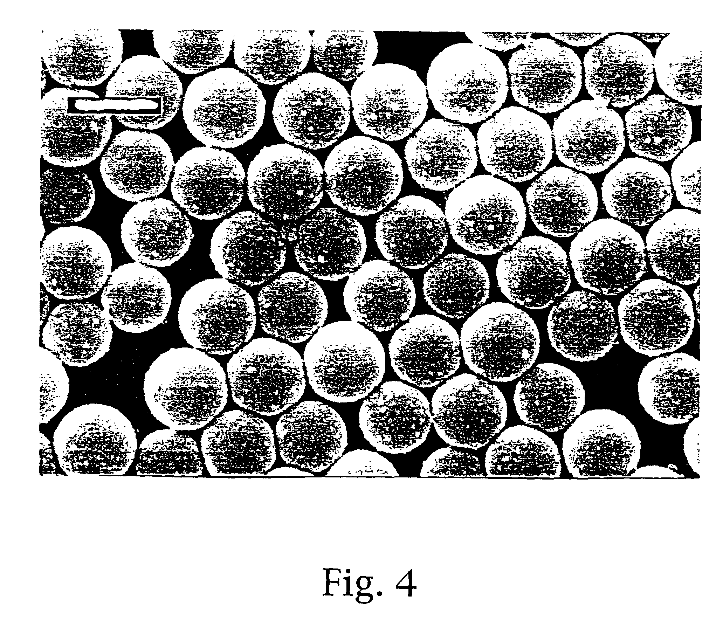

Les mesures effectuées montrent que les microsphères ont un diamètre moyen de 1,4 micromètre avec un écart-type de 5 %. Le taux de fer est de 12,5 %. Une photographie ci-jointe pour un agrandissement de 20 000 fois montre l'homogénéité remarquable des microsphères obtenues (figure 4).

## Revendications

1. Procédé de préparation par polymérisation en dispersion d'un latex magnétique monodisperse calibré formé d'une dispersion aqueuse de microsphères magnétisables constituées d'une matrice de polymères hydrophobes dérivés d'au moins un monomère vinylique et de charges magnétisables, lesdites microsphères possédant une distribution granulométrique calibrée, une taille comprise entre 0,5 et 5 micromètres, caractérisé en ce que l'on fait réagir en une seule étape une solution organique comprenant au moins un monomère vinylique polymérisable, un solvant organique, qui est solvant pour les monomères et non solvant pour le polymère formé, des charges magnétisables sous forme de ferrofluide, un système stabilisant pour le polymère formé, en présence d'un initiateur de polymérisation organosoluble et en ce que l'on récupère ledit latex magnétique.

2. Procédé de préparation selon la revendication 1, caractérisé en ce que le solvant est polaire.

3. Procédé de préparation selon la revendication 2, caractérisé en ce que le solvant est un solvant alcoolique ou éther ou un mélange eau-alcool ou éther-alcool.

4. Procédé de préparation selon la revendication 2 ou 3, caractérisé en ce que les monomères sont choisis dans le groupe constitué par les monomères vinylaromatiques, l'acide acrylique, l'acide méthacrylique, les acrylates et méthacrylates d'alkyle dont le groupement alkyle possède de 1 à 10 atomes de carbone, les hydroxyalkylacrylates et méthacrylates, l'acrylamide et le méthacrylamide, les monomères vinyliques présentant un groupement attracteur, et les diènes.

5. Procédé de préparation selon la revendication 1, caractérisé en ce que le système stabilisant est un polymère choisi dans le groupe constitué par la poly(vinyl pyrrolidone), les homopolymères acryliques ou méthacryliques, les copolymères statistiques ou séquencés de monomères choisis dans le groupe constitué par le styrène ou les dérivés du styrène, le butadiène d'une part, l'acide méthacrylique, l'acide acrylique d'autre part.

6. Procédé de préparation selon la revendication 1, caractérisé en ce que le solvant est apolaire.

7. Procédé de préparation selon la revendication 6, caractérisé en ce que le solvant est choisi dans le groupe constitué par l'hexane, le cyclohexane.

8. Procédé de préparation selon la revendication 6, caractérisé en ce que les monomères sont choisis dans le groupe constitué par les acrylates ou méthacrylates d'alkyle, les dérivés styréniques, les diènes.

9. Procédé de préparation selon la revendication 6, caractérisé en ce que le système stabilisant est un polymère choisi dans le groupe constitué par les polyacrylates ou méthacrylates, les copolymères séquencés ou statistiques de monomères choisis dans le groupe constitué par les oléfines, les dérivés styrèniques, le butadiène, les dérivés du butadiène, les esters vinyliques d'une part, les dérivés acrylates ou méthacrylates, les alkylsiloxanes d'autre part.

10. Procédé de préparation selon la revendication 1, caractérisé en ce que la solution comprend un costabilisant ionique.

11. Procédé de préparation selon la revendication 10, caractérisé en ce que le costabilisant est choisi dans le groupe constitué par le dioctylsulfosuccinate de sodium, le chlorure de méthyltricaprylammonium.

12. Procédé de préparation selon la revendication 1, caractérisé en ce que l'initiateur de polymérisation est choisi dans le groupe constitué par l'AIBN ou 2,2'-azobis(isobutyronitrile), l'ACPA ou 4,4'-azobis(acide 4-cyanopentanoique), l'AMBN ou 2,2'-azobis(2-méthylbutyronitrile), le BPO ou peroxyde de benzoyle, l'ADVN ou 2,2'-azobis(2,4-diméthylvaleronitrile).

13. Procédé de préparation selon la revendication 1, caractérisé en ce que la solution comprend en partie en poids :
| | |
|---|---|
| solvant organique | 45 à 90 |
| monomère | 5 à 30 |
| ferrofluide | 2 à 30 |
| système stabilisant | 2 à 10 |
| costabilisant | 0,1 à 1 |
| initiateur | 0,1 à 1 |

## Claims

1. Process for the preparation by dispersion polymerization of a calibrated monodisperse magnetic latex made up of an aqeous dispersion of magnetizable microspheres composed of a matrix of hydrophobic polymers derived from at least one vinyl monomer and of magnetizable fillers, the said microspheres having a calibrated particle size distribution and a size of between 0.5 and 5 micrometres, characterized in that an organic solution including at least one polymerizable vinyl monomer, an organic solvent, which is a solvent for the monomers and not a solvent for the polymer formed, magnetizable fillers in ferrofluid form and a stabilizing system for the polymer formed is reacted in a single step in the presence of an organosoluble polymerization initiator and in that the said magnetic latex is recovered.

2. Process of preparation according to Claim 1, characterized in that the solvent is polar.

3. Process of preparation according to Claim 2, characterized in that the solvent is an alcoholic or ether solvent or a water-alcohol or ether-alcohol mixture.

4. Process of preparation according to Claim 2 or 3, characterized in that the monomers are chosen from the group consisting of vinylaromatic monomers, acrylic acid, methacrylic acid, alkyl acrylates and methacrylates, the alkyl group of which has from 1 to 10 carbon atoms, hydroxyalkyl acrylates and methacrylates, acrylamide and methacrylamide, vinyl monomers which have an attracting group and dienes.

5. Process of preparation according to Claim 1, characterized in that the stabilizing system is a polymer chosen from the group consisting of poly(vinyl pyrrolidone), acrylic or methacrylic homopolymers and random or block copolymers of monomers chosen from the group consisting of, on the one hand, styrene or styrene derivatives and butadiene and, on the other hand, methacrylic acid and acrylic acid.

6. Process of preparation according to Claim 1, characterized in that the solvent is apolar.

7. Process of preparation according to Claim 6, characterized in that the solvent is chosen from the group consisting of hexane and cyclohexane.

8. Process of preparation according to Claim 6, characterized in that the monomers are chosen from the group consisting of alkyl acrylates or methacrylates, styrene derivatives and dienes.

9. Process of preparation according to Claim 6, characterized in that the stabilizing system is a polymer chosen from the group consisting of polyacrylates or methacrylates, block or random copolymers of monomers chosen from the group consisting of, on the one hand, olefins, styrene derivatives, butadiene, butadiene derivatives and vinyl esters and, on the other hand, acrylate or methacrylate derivatives and alkylsiloxanes.

10. Process of preparation according to Claim 1, characterized in that the solution includes an ionic costabilizer.

11. Process of preparation according to Claim 10, characterized in that the costabilizer is chosen from the group consisting of sodium dioctylsulphosuccinate and methyltricaprylammonium chloride.

12. Process of preparation according to Claim 1, characterized in that the polymerization initiator is chosen from the group consisting of AIBN, or 2,2'-azobis(isobutyronitrile), ACPA, or 4,4'-azobis(4-cyanopentanoic acid), AMBN, or 2,2'-azobis(2-methylbutyronitrile), BPO, or benzoyl peroxide, and ADVN, or 2,2'-azobis(2,4-dimethylvaleronitrile).

13. Process of preparation according to Claim 1, characterized in that the solution includes, in parts by weight:
| | |
|---|---|
| organic solvent | 45 to 90 |
| monomer | 5 to 30 |
| ferrofluid | 2 to 30 |
| stabilizing system | 2 to 10 |
| costabilizer | 0.1 to 1 |
| initiator | 0.1 to 1 |

## Patentansprüche

1. Verfahren zur Herstellung eines monodispersen magnetischen kalibrierten Latex durch Polymerisation in Dispersion aus einer wässrigen Dispersion von magnetisierbaren Mikrospheren, bestehend aus einer Matrix von hydrophoben Polymeren aus mindestens einem Vinylmonomeren und magnetisierbaren Zusätzen, wobei die Mikrospheren in granulometrischer Verteilung eine kalibrierte Größe von zwischen 0,5 und 5 µm aufweisen, dadurch gekennzeichnet, daß man in einer Stufe eine organische Lösung mit Gehalt an mindestens einem polymerisierbaren Vinylmonomeren, einem organischen Lösungsmittel, das ein Lösungsmittel für die Monomeren und ein Nichtlösungsmittel für das gebildete Polymere ist, magnetisierbaren Zusätzen in Form von Eisenfluid, einem das gebildete Polymere stabilisierendem System, in Gegenwart eines organolöslichen Polymerisationsinitators umsetzt und die magnetische Latex gewinnt.

2. Verfahren nach Anspruch 1, dadurch gekennzeichnet, daß das Lösungsmittel polar ist.

3. Verfahren nach Anspruch 2, dadurch gekennzeichnet, daß das Lösungsmittel ein alkoholisches Lösungsmittel oder Äther oder eine Mischung Wasser/Alkohol oder Äther/Alkohol ist.

4. Verfahren nach einem der Ansprüche 2 oder 3, dadurch gekennzeichnet, daß man als Monomere vinylaromatische Monomere, Acrylsäure, Methacrylsäure, Alkylacrylate oder -methacrylate, deren Alkylgruppe 1 bis 10 Kohlenstoffatome aufweist, Hydroxyalkyacrylate und -methacrylate, Acrylamid und Methacrylamid, vinylische Monomere mit olektronenanziehenden Gruppen und/oder Diene auswählt.

5. Verfahren nach Anspruch 1, dadurch gekennzeichnet, daß das stabilisierende System eines der Polymeren Poly (vinylpyrrolidon), acrylische oder methacrylische Homopolymere, statistische oder regelmäßige Copolymere von Styrol oder Styrolderivaten, Butadien einerseits und Acryl-, Methacrylsäure andererseits, ist.

6. Verfahren nach Anspruch 1, dadurch gekennzeichnet, daß das Lösungsmittel apolar ist.

7. Verfahren nach Anspruch 6, dadurch gekennzeichnet, daß das Lösungsmittel Hexan oder Cyclohexan ist.

8. Verfahren nach Anspruch 6, dadurch gekennzeichnet, daß die Monomeren Alkylacrylate oder Alkylmethacrylate, Styrolderivate und/oder Diene sind.

9. Verfahren nach Anspruch 6, dadurch gekennzeichnet, daß das stabilisierende System eines der Polymeren Polyacrylate oder- methacrylate, regelmäßige oder statistische Copolymere der Monomeren Olefine, Styrolderivate, Butadien, Butadienderivate, Vinylester auf der einen Seite und Acrylate- oder Methacrylderivate, Alkylsiloxane auf der anderen Seite, ist.

10. Verfahren nach Anspruch 1, dadurch gekennzeichnet, die Lösung einen ionischen Costabilisator aufwcist.

11. Verfahren nach Anspruch 10, dadurch gekennzeichnet, daß der Costabilisator Natriumdioctylsulfosuccinat und/oder Methyltricaprylammoniumchlorid ist.

12. Verfahren nach Anspruch 1, dadurch gekennzeichnet, daß der Polymerisationsinitiator AIBN oder 2,2'-Azobis (isobutyronitril), ACPA oder 4,4'-Azobis(4-cyanopentansäure), AMBN oder 2,2'-Azobis(2-methylbutyronitril), BPO oder Benzolperoxid, ADVN oder 2,2'-Azobis(2,4-dimethylvaleronitril) ist.

13. Verfahren nach Anspruch 1, dadurch gekonnzeichnet, daß die Lösung in Gewichtsteilen enthält:
| | |
|---|---|
| Organisches Lösungsmittel | 45 bis 90 |
| Monomer | 5 bis 30 |
| Ferrofluid | 2 bis 30 |
| Stabilisierendes System | 2 bis 10 |
| Costabilisator | 0,1 bis 1 |
| Initiator | 0,1 bis 1 |
